# EUROPEAN PATENT APPLICATION

(11) **EP 2 422 812 A1**
(43) Date of publication of application: **29.02.2012**
(21) Application number: 11183631.8
(22) Date of filing: 20.02.2004
(51) Int. Cl.: A61K 39/395, C07K 16/18

(54) **Methods for preventing and treating tissue damage associated with ischemia-reperfusion injury**

(30) Priority: 21.02.2003 US 449069 P
(62) Divisional of application: 04713419.2
(71) Applicant: Genentech, Inc., South San Francisco, CA 94080 (US)
(72) Inventor: Fung, Sek Chung, Houston, TX 77025 (US)
(74) Representative: Evenson, Jane Harriet

(57) **Abstract**

A method for preventing or treating tissue damage associated with ischemia-reperfusion injury and thoraco-abdom-inal aortic aneurysm (TAAA) repair by administering a tissue damage preventing or treating amount of a complement inhibitor to a patient likely to suffer from or suffering from tissue damage associated with ischemia-reperfusion injury or TAAA repair. The complement inhibitors are preferably antibodies that bind to and inhibit complement proteins involved in the formation of the membrane attach complex, preferably antibodies that inhibit MBL, MASP1, MASP2, and MASP3 in the lectin pathway. The complement inhibitors can be used alone or in combination to decrease the morbidity and mortality caused by tissue damage associated with ischemia-reperfusion injury or TAAA repair

## Description

### CROSS REFERENCE TO RELATED APPLICATIONS

This application claims priority to U.S. Provisional Application Serial No. 60/449,069, filed February 2I, 2003, the disclosure of which is incorporated herein by this reference.

### BACKGROUND OF THE INVENTION

### Field of the Invention

This invention relates generally to methods and compositions for the prevention and treatment of tissue damage and particularly to methods and compositions for the prevention or treatment of tissue damage associated with ischemia-reperfusion injury.

### Description of the Prior Art

### Immune System Complement

The immune system protects the body against pathogenic bacteria, viruses, parasites and other harmful organisms. The immune system is divided into two components, the humoral system and the cellular system. Generally, the humoral system includes the complement system and the production of antibodies to defend against pathogens. The complement system, or simply complement, involves the production of proteins that assist the antibodies in the host defense. The complement system is an integrated part of innate immunity. Complement can discriminate not only between "self" and "non-self' but also between formal self' and "altered self" Complement is a group of at least 30 surface-bound and soluble proteins. The activity of certain soluble proteins is destroyed by heating serum at 56°C for 30 minutes. Complement proteins are involved in the opsonization of microorganisms for phagocytosis, direct killing of microorganisms by lysis, chemotactic attraction of leukocytes to sites of inflammation, activation of leukocytes, and processing of immune complexes.

Complement proteins work in a cascade wherein the binding or activation of one protein promotes the binding or activation of the next protein in the cascade. Activation of the cascade leads to release of biologically active small peptides called anaphylatoxins (C3a, C4a and the most potent C5a) contributing to the inflammatory reaction, and eventually in the formation of a membrane attack complex (C5b-9) that may lyse the target cell. Different complement molecules are synthesized by different cell types, e.g. fibroblasts and intestinal epithelial cells make C1, while most of the components are synthesixed in the liver.

The components and mechanism of the complement system are well known. Basically, there are three complement pathways, the classical pathway, the lectin pathway, and the alternative pathway. The classical pathway is triggered primarily by immune complexes containing antigen and IgG or IgM, but also by other agents like C-reactive protein. The lectin pathway is triggered by binding af mannose binding lectin (MBL) or ficolins to carbohydrate structures (e.g., mannan) on foreign surfaces. The alternative pathway is activated principally by repeating polysaccharides and other polymeric structures such as those found on bacteria.

The classical pathway is activated when the globular domains of C1q (part of the Clqrs complex) bind to the Fc fragments of IgM or multiple molecules of IgG. In the presence of calcium ions, this binding causes the autocatalytic activation of two C1r molecules. The C1r molecules activate two molecules of C1s. C1s is a serine protease that cleaves C4a from C4b. C4b immediately binds to adjacent proteins or carbohydrates on the surface of the target cell and then binds to C2 in the presence of magnesium ions. C1s cleaves C2b from this complex, yielding the classical pathway C3 convertase, C4b2a. The C3 convertase cleaves many hundreds of molecules of C3 into C3a and C3b. Some molecules of C3b will bind back to C4b2a to yield the classical pathway C₅ convertase, C4b2a3b. C5 convertase cleaves C5 into C5a and C5b. C5b binds to the surface of the cell, initiating the formation of the membrane attack complex (MAC).

The "lectin pathway" is similar to the classical pathway except it is initiated by the calcium-dependent lectin MBL that binds to terminal mannose groups on the surface of bacteria. MBL is an oligomer of subunits composed of identical polypeptide chains each of which contains a cysteine-rich domain, a collagen-like domain, a neck domain, and a carbohydrate-recognition domain. MBL as defined includes several sizes of these oligomers. MBL is analogous to Clq. When MBL binds to its target, e.g., mannose or N-acetylglucosamine (GlcNAc)), the interaction leads to the activation of three serine proteases known as MASP1, MASP2, and MASP3 (mannose-binding lectin-associated serine protease), which are analogous to C1r and C1s. Among them, MASP2 is responsible for the cleavage of C4 into C4b and C4a, and C2 into C2a and C2b. C2a and C4b then bind to form the classical pathway C3 convertase. From this point onward, the lectin pathway is identical to the classical pathway.

The alternative complement pathway involves an amplification loop utilizing C3b produced by the classical pathway and the lectin pathway. Some molecules of C3b generated by the classical pathway C3 convertase are funneled into the alternative pathway. Surface-bound C3b binds Factor B to yield C3bB, which becomes a substrate for Factor D. Factor D is a serine esterase that cleaves the Ba fragment, leaving C3bBb bound to the surface of the target cell. C3bBb is stabilized by properdin (P), forming the complex C3bBbP, which acts as the alternative pathway C3 convertase. This C3 convertase participates in an amplification loop to cleave many C3 molecules, resulting in the deposition of C3b molecules on the target cell. Some of these C3b molecules bind back to C3bBb to form C3bBb3b, the alternative pathway C5 convertase. C5 convertase cleaves C5 into C5a and C5b. C5b binds to the surface of the cell to initiate the formation of the membrane attack complex.

The classical, lectin, and alternative complement pathways all end with the formation of C5 convertase. C5 convertase leads to the assembly of the membrane attack complex (C5b6789n) via the lytic pathway. Components C5-C8 attach to one another in tandem and promote the insertion of one or more monomers of C9 into the lipid bilayer of the target cell. This insertion leads to the formation of pores that cause calcium influx with subsequent cellular activation of nucleated cells or cell lysis and death if the attack is sufficiently strong.

Complement activation has been shown to be a factor in the pathogenesis of several diseases associated with local or systemic inflammation. Kyriakides, et al. demonstrated that the complement alternative pathway plays a significant role in acid aspiration injury (Membrane attack complex of complement and neutrophils mediate the injury of acid aspiration. J. Appl. Physiol. 87(6): 2357-2361, 1999 and Sialyl Lewis^{x} hybridized complement receptor type 1 moderates acid aspiration injury. Am J Physiol Lung Cell Mol Physiol 281: L1494-L1499, 2001). US Patent No. 6,492,403 discloses a method for treating the symptoms of an acute or chronic disorder mediated by the classical pathway of the complement cascade using furanyl and thienyl amidines and guanidines. US Patent No. 6,458,360 discloses a soluble recombinant fused protein comprising a polypeptide that contains a recognition site for a target molecule, such as a complement receptor site, and is joined to the N-terminal end of an immunoglobulin chain that is useful for inhibiting complement activation or complement-dependent cellular activation in mammals. WO0112212 discloses inhibitors of the lectin complement pathway and their use. WO0035483 discloses methods and products for regulating lectin complement pathway associated complement activation.

### Ischemia and Reperfusion

Ischemia-reperfusion is the interruption of blood flow to bodily tissue and the subsequent and often abrupt restoration of blood flow to the tissue. While restoration of blood flow following ischemia is essential to preserve functional tissue, the reperfusion itself is known to be harmful to the tissue. Both ischemia and reperfusion are known to be important contributors to tissue necrosis.

Several mechanisms appear to play a causative role in the generation of tissue damage associated with ischemia-reperfusion injury. To some extent, most of these mechanisms involve neutrophils. The infiltration of neutrophils into ischemic tissue is responsible for much of the tissue damage associated with ischemia-reperfusion injury. Neutrophils contain an NADPH oxidase that reduces molecular oxygen to a superoxide anion. Neutrophil accumulation initiated by reperfusion is significantly reduced by pretreatment with xanthine oxidase inhibitors, oxygen radical scavengers, or iron chelators. This suggests that reactive oxygen metabolites play a role in the recruitment of neutrophils into post ischemic tissue and that xanthine oxidase derived oxidants, produced in epithelial and endothelial cells, initiate the production and release of proinflammatory agents that subsequently attract and activate neutrophils. Further, the neutrophil membrane glycoprotein CD18 has been shown to play an important role in mediating neutrophil adhesion to microvascular endothelium. Monoclonal antibodies directed against the CD18 receptor inhibit the chemotaxis, aggregation, and adherence of neutrophils to capillary endothelium. Use of this receptor specific antibody has reduced reperfusion injury as effectively as neutropenia induced by radiation, filters, or anti-neutrophil antibodies. Therefore, neutrophil adherence to the microvascular endothelium appears to be an essential step in neutrophil-mediated reperfusion injury and the tissue damage associated with ischemia-reperfusion injury.

### Thoraco-Abdominal Aortic Aneurysms and Their Repair

An aneurysm that involves the thoracic and abdominal aorta is called a thoraco-abdominal aortic aneurysm (TAAA). Historically, patients that experienced a TAAA and are subsequently undergoing TAAA repair have relatively high morbidity and mortality rates, particularly the risk of paraplegia. Paraplegia risk is as high as 40 percent depending on the extent of tissue damage and cause of the aneurysm. Paraplegia and other neurological complications that result from tissue damage associated with TAAA repair are often due to spinal cord ischemia (oxygen deprivation and inadequate waste removal due to reduced perfusion) and systemic inflammation.

Known methods for preventing or treating tissue damage associated with TAAA repair are based upon careful surgical and anesthesia procedures that reduce the morbidity and mortality rate associated with TAAA repair (Thoracoabdominal Aortic Aneurysm Repair in High Risk Cardiac Patients: A Modified Grafting Technique. Angiology, 7:118-122, 1998). These methods, however, have met with limited success because even a technically successful procedure may still be complicated by multi-organ dysfunction and other problems that cause morbidity and mortality. There is, therefore, a need for new methods and compositions for preventing or treating tissue damage associated with ischemia-reperfusion injury and TAAA repair.

### SUMMARY OF THE INVENTION

It is, therefore, an object of the present invention to provide methods and compositions for preventing or treating tissue damage associated with ischemia-reperfusion injury.

It is a further object of the present invention to provide methods and compositions for preventing or treating tissue damage associated with TAAA repair.

It is another object of the invention to decrease the morbidity and mortality caused by tissue damage associated with ischemia-reperfusion injury and TAAA repair.

These and other objects are achieved using a novel method for preventing or treating tissue damage associated with ischemia-reperfusion injury and with TAAA repair. The method comprises administering a tissue damaging preventing or treating amount of one or more complement inhibitors to a patient likely to suffer from or suffering from tissue damage associated with ischemia-reperfusion injury or with TAAA repair. The complement inhibitor can be any known complement inhibitor but is preferably an antibody or functionally equivalent fragment thereof that binds to and inhibits complement proteins in the lectin pathway. The antibody or antibody fragment inhibits the action of proteins that are involved in the complement pathways, e.g., C3a, C5a, MBL MASP, and the membrane attack complex (MAC), and inhibits or prevents damage to tissues and cells when complement is activated in response to ischemia-reperfusion injury or ischemia-reperfusion injury following TAAA repair in a patient.

Other and further objects, features and advantages of the present invention will be readily apparent to those skilled in the art.

### BRIEF DESCRIPTION OF THE DRAWINGS

Figure 1 shows data relating to the complement analysis for MBL-deficient TAAA patients.

Figure 2 shows data relating to initial complement pathway activation products.

Figure 3 shows data relating to activation of C3 and the terminal complement pathway.

Figure 4 shows data relating to the cytokines and chemokines IL-1β, TNFα, and TL-8.

Figure 5 shows data relating to cytokines and chemokines IL-6 and IL-10.

Figure 6 shows data relating to neutrophil degradation products.

### DETAILED DESCRIPTION OF THE INVENTION

### Definitions

The term "patient" means a human or other animal likely to suffer from or suffering from tissue damage associated with TAAA repair, including bovine, porcine, canine, feline, equine, avian, and ovine animal. Preferably, the patient is a human.

The term "parenterally" means administration by intravenous, subcutaneous, intramuscular, or intraperitoneal injection.

The term "in conjunction" means that different complement inhibitors are administered to the patient (1) separately at the same or different frequency using the same or different administration routes or (2) together in a pharmaceutically acceptable composition.

The term "functionally equivalent fragments" means antibody fragments that bind to components of the complement system and inhibit complement activation in substantially the same manner as the complete antibody. Unless otherwise specified, all antibodies described herein are defined to include their functionally equivalent fragments.

This invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise, e.g., reference to "an antibody" includes a plurality of such antibodies.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, devices, and materials are described herein.

All patents and publications mentioned herein are incorporated herein by reference to the extent allowed by law for the purpose of describing and disclosing the compounds and methodologies reported therein that might be used with the present invention. However, nothing herein is to be construed as an admission that the invention is not entitled to antedate such disclosure by virtue of prior invention.

### The Invention

In one aspect, the present invention provides a method for preventing and treating tissue damage associated with ischemia-reperfusion injury. The method comprises administering a tissue damage preventing or treating amount of one or more complement inhibitors to a patient likely to suffer from or suffering from tissue damage associated with ischemia-reperfusion injury. The invention is based upon the discovery that the complement components of the immune system play a critical role in the development of tissue damage during ischemia-reperfusion injury and that methods and compositions for inhibiting or preventing complement activation can be used to prevent or treat such tissue damage. The methods and compositions are useful for decreasing the morbidity and mortality for patients susceptible to or suffering from tissue damage associated with ischemia-reperfusion injury.

In another aspect, the present invention provides a method for preventing and treating tissue damage associated with thoraco-abdominal aortic aneurysm (TAAA) repair. The method comprises administering a tissue damage preventing or treating amount of one or more complement inhibitors to a patient likely to suffer from or suffering from tissue damage associated with TAAA repair. The invention is based upon the discovery that the complement components of the immune system play a critical role in the development of tissue damage during TAAA repair and that methods and compositions for inhibiting or preventing complement activation can be used to prevent or treat such tissue damage. The methods and compositions are useful for decreasing the morbidity and mortality for patients susceptible to or suffering from tissue damage associated with TAAA repair.

The complement inhibitors of the present invention are any molecule known to inhibit complement activation in a patient. Generally, these inhibitors are small organic molecules, peptides, proteins, antibodies, antibody fragments, or other molecules that function as complement inhibitors. Useful complement inhibitors include compstatin and its functional analogs (inhibits C3), C1 Inhibitor (covalently binds C1r and C1s), sCR1 and its analogues (dissociate all C3 convertases), anti-C5 antibodies (block C5 activation), anti-C5a and anti-C5a receptor antibodies and small-molecule drugs (inhibit C5a signaling pathway), anti-C3a and anti-C3a receptor antibodies and small-molecule drugs (inhibit C3a signaling pathway), anti-C6, 7, 8, or 9 antibodies (inhibit the formation or function of MAC), anti-properdin antibodies (destabilize C3 and C5 convertases in the alternative pathway), and a fusion protein Membrane Cofactor Protein (cofactor for Factor I mediated C3b and C4b cleavage) and Decay Accelerating Factor (DAF) (accelerates decay of all C3 convertases). Other useful inhibitors include clusterin (inhibits C1), CD59 (membrane attack complex inhibitor), C4bp (accelerates decay of classical pathway C3 convertase (C4b2a)), Factor H (accelerates decay of alternative pathway C3 convertase (C3bBb)), Factor I (proteolytically cleaves and inactivates C4b and C3b (cofactors are required)), Carboxypeptidase N (removes terminal arginine residues from C3a, C5a), vitronectin (S Protein) (binds C5b-7 complex and prevents membrane insertion), SP-40 (modulates membrane attack complex formation), CD59 (inhibits lysis of bystander cells), and Homologous Restriction Factor (HRF) (inhibits bystander lysis, C8 and C9 interactions).

Preferably, the complement inhibitors are antibodies or functionally equivalent fragments that bind to and inhibit one or more of the proteins that function in the complement cascade, e.g., C1, C3, C5, Factor D, or their components and protease cleavage products. The antibodies bind to a selected complement protein in the complement cascade and inhibit or prevent complement activation during TAAA repair. In one embodiment, the complement inhibitor is an anti-C5 antibody or functionally equivalent fragment thereof that binds to C5 and inhibits its action in the complement cascade. The antibody can also be an anti-C5a or anti-C5b antibody that binds to these proteins and inhibits their action in the complement cascade. Similarly, the complement inhibitor is an anti-Factor D antibody or functionally equivalent fragment thereof that binds to Factor D and inhibits its action in the complement cascade. The antibodies can be a polyclonal or monoclonal antibodies but are preferably monoclonal antibodies.

In a preferred embodiment, the complement inhibitors are compounds that inhibit the lectin complement pathway. Such inhibitors include anti-MBL antibodies and their functionally equivalent fragments, anti-MASP antibodies and their functionally equivalent fragments, anti-MASP2 antibodies and their functionally equivalent fragments, anti-MASP3 antibodies and their functionally equivalent fragments, anti-MBL complex antibodies (antibodies that bind to the complex formed by MBL, MASP1, MASP2, and MASP3) and their functionally equivalent fragments, mannan binding lectin receptor antagonists (such as legume derived lectins that bind MBL), keratin binding molecules, anti-keratin antibodies and their functionally equivalent fragments, MASP binding peptides, MASP2 binding peptides, and MASP3 binding peptides.

In one embodiment two or more complement inhibitors are administered to a patient in conjunction to prevent and treat tissue damage associated with ischemia-reperfusion injury, particularly ischemia-reperfusion injury associated with TAAA repair. For example, an anti-MBL antibody is administered in conjunction with another complement inhibitor to prevent or treat such tissue damage. Various combinations of anti-MBL antibodies and their functionally equivalent fragments, anti-MASP1 antibodies and their functionally equivalent fragments, anti-MASP2 antibodies and their functionally equivalent fragments, anti-MASP3 antibodies and their functionally equivalent fragments, anti-MBL complex antibodies and their functionally equivalent fragments, anti-Factor D antibodies and their functionally equivalent fragments, and anti-properdin antibodies and their functionally equivalent fragments are preferred.

Methods for producing antibodies, including polyclonal, monoclonal, monovalent, humanized, human, bispecific, and heteroconjugate antibodies, are well known to skilled artisans.

### Polyclonal Antibodies

Polyclonal antibodies can be produced in a mammal by injecting an immunogen alone or in combination with an adjuvant. Typically, the immunogen is injected in the mammal using one or more subcutaneous or intraperitoneal injections. The immunogen may include the polypeptide of interest or a fusion protein comprising the polypeptide and another polypeptide known to be immunogenic in the mammal being immunized. The immunogen may also include cells expressing a recombinant receptor or a DNA expression vector containing the receptor gene. Examples of such immunogenic proteins include, but are not limited to, keyhole limpet hemocyanin, serum albumin, bovine thyroglobulin, and soybean trypsin inhibitor. Examples of adjuvants include, but are not limited to, Freund's complete adjuvant and MPL-TDM adjuvant (monophosphoryl Lipid A, synthetic trehalose dicorynomycolate). The immunization protocol may be selected by one skilled in the art without undue experimentation.

### Monoclonal Antibodies

Monoclonal antibodies can be produced using hybridoma methods such as those described by Kohler and Milstein, Nature, 256:495 (1975). In a hybridoma method, a mouse, hamster, or other appropriate host mammal, is immunized with an immunogen to elicit lymphocytes that produce or are capable of producing antibodies that will specifically bind to the immunogen. Alternatively, the lymphocytes may be immunized in vitro. The immunogen will typically include the polypeptide of interest or a fusion protein containing such polypeptide. Generally, peripheral blood lymphocytes ("PBLs") cells are used if cells of human origin are desired. Spleen cells or lymph node cells are used if cells of non-human mammalian origin are desired. The lymphocytes are then fused with an immortalized cell line using a suitable fusing agent, e.g., polyethylene glycol, to form a hybridoma cell (Goding, Monoclonal Antibodies: Principles and Practice, pp 59-103 (Academic Press, 1986)). Immortalized cell lines are usually transformed mammalian cells, particularly rodent, bovine, or human myeloma cells. Usually, rat or mouse myeloma cell lines are employed. The hybridoma cells may be cultured in a suitable culture medium that preferably contains one or more substances that inhibit the growth or survival of the unfused immortalized cells. For example, if the parental cells lack the enzyme hypoxanthine guanine phosphoribosyl transferase (HGPRT), the culture medium for the hybridomas typically will include hypoxanthine, aminopterin, and thymidine (HAT medium). The HAT medium prevents the growth of HGPRT deficient cells.

Preferred immortalized cell lines are those that fuse efficiently, support stable high level expression of antibody by the selected antibody producing cells, and are sensitive to a medium such as HAT medium. More preferred immortalized cell lines are murine myeloma lines such as those derived from MOPC-21 and MPC-11 mouse tumors available from the Salk Institute Cell Distribution Center, San Diego, Calif. USA, and SP2/0 or X63-Ag8-653 cells available from the American Type Culture Collection, Rockville, Md. USA. Human myeloma and mouse-human heteromyeloma cell lines also have been described for use in the production of human monoclonal antibodies (Kozbor, J. Immunol. 133:3001 (1984); Brodeur et al., Monoclonal Antibody Production Techniques and Applications, pp. 51-63 (Marcel Dekker, Inc., New York, 1987)). The mouse myeloma cell line NS0 may also be used (European Collection of Cell Cultures, Salisbury, Wiltshire UK). Human myeloma and mouse-human heteromyeloma cell lines, well known in the art, can also be used to produce human monoclonal antibodies.

The culture medium used for culturing hybridoma cells can then be assayed for the presence of monoclonal antibodies directed against the polypeptide of interest. Preferably, the binding specificity of monoclonal antibodies produced by the hybridoma cells is determined by immunoprecipitation or by an in vitro binding assay, e.g., radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem, 107:220 (1980).

After the desired hybridoma cells are identified, the clones may be subcloned by limiting dilution procedures and grown by standard methods. Suitable culture media for this purpose include Dulbecco's Modified Eagle's Medium and RPMI-1640 medium. Alternatively, the hybridoma cells may be grown in vivo as ascites in a mammal.

The monoclonal antibodies secreted by the subclones are isolated or purified from the culture medium or ascites fluid by conventional immunoglobulin purification procedures such as protein A-Sepharose, hydroxylapatite chromatography, gel electrophoresis, dialysis, or affinity chromatography.

The monoclonal antibodies may also be produced by recombinant DNA methods, e.g., those described in U.S. Pat No. 4,816,567. DNA encoding the monoclonal antibodies of the invention can be readily isolated and sequenced using conventional procedures, e.g., by using oligonucleotide probes that are capable of binding specifically to genes encoding the heavy and light chains of murine antibodies (Innis M. et al. In "PCR Protocols. A Guide to Methods and Applications", Academic, San Diego, CA (1990), Sanger, F.S, et al. Proc. Nat. Acad. Sci. 74:5463-5467 (1977)). The hybridoma cells described herein serve as a preferred source of such DNA. Once isolated, the DNA may be placed into expression vectors. The vectors are then transfected into host cells such as simian COS cells, Chinese hamster ovary (CHO) cells, or myeloma cells that do not otherwise produce immunoglobulin protein. The recombinant host cells are used to produce the desired monoclonal antibodies. The DNA also may be modified, for example, by substituting the coding sequence for human heavy and light chain constant domains in place of the homologous murine sequences or by covalently joining the immunoglobulin coding sequence to all or part of the coding sequence for a non-immunoglobulin polypeptide. Such a non immunoglobulin polypeptide can be substituted for the constant domains of an antibody or can be substituted for the variable domains of one antigen combining site of an antibody to create a chimeric bivalent antibody.

Monovalent antibodies can be produced using the recombinant expression of an immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking. Similarly, in vitro methods can be used for producing monovalent antibodies. Antibody digestion can be used to produce antibody fragments, preferably Fab fragments, using known methods.

Antibodies and antibody fragments can be produced using antibody phage libraries generated using the techniques described in McCafrerty, et al., Nature 348:552-554 (1990). Clackson, et al., Nature 352:624-628 (1991) and Marks, et al., J. Mol. Biol. 222:581-597 (1991) describe the isolation of murine and human antibodies, respectively, using phage libraries. Subsequent publications describe the production of high affinity (nM range) human antibodies by chain shuffling (Marks, et al., Bio/Technology 10:779-783 (1992)), as well as combinatorial infection and in vivo recombination as a strategy for constructing very large phage libraries (Waterhouse, et al., Nuc. Acids. Res. 21:2265-2265 (1993)). Thus, these techniques are viable alternatives to traditional Monoclonal antibody hybridoma techniques for isolation of monoclonal antibodies. Also, the DNA may be modified, for example, by substituting the coding sequence for human heavy-chain and light-chain constant domains in place of the homologous murine sequences (U.S. Pat. No. 4,816,567; Morrison, et al., Proc. Nat. Acad. Sci. USA 81:6851 (1984)), or by covalently joining to the immunoglobulin coding sequence all or part of the coding sequence for a non-immunoglobulin polypeptide. Typically, such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

Antibodies can also be produced using use electrical fusion rather than chemical fusion to form hybridomas. This technique is well established. Instead of fusion, one can also transform a B-cell to make it immortal using, for example, an Epstein Barr Virus, or a transforming gene "Continuously Proliferating Human Cell Lines Synthesizing Antibody of Predetermined Specificity," Zurawaki, V. R. et al, in "Monoclonal Antibodies," ed. by Kennett R. H. et al, Plenum Press, N.Y. 1980, pp 19-33.

### Humanized Antibodies

Humanized antibodies can be produced using the method described by Winter in Jones et al., Nature, 321:522-525 (1986); Riechmann et al., Nature, 332:323-327 (1988); and Verhoeyen et al., Science, 239:1 534-1536 (1988). Humanization, is accomplished by substituting rodent CDRs or CDR sequences for the corresponding sequences of a human antibody. Generally, a humanized antibody has one or more amino acids introduced into it from a source that is non-human. Such "humanized" antibodies are chimeric antibodies wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies. Humanized forms of non-human (e.g., murine or bovine) antibodies are chimeric immunoglobulins, immunoglobulin chains, or immunoglobulin fragments such as Fv, Fab, Fab', F(ab')₂, or other antigen-binding subsequences of antibodies that contain minimal sequence derived from non-human immunoglobulin. Humanized antibodies include human immunoglobulins (recipient antibody) wherein residues from a complementary determining region (CDR) of the recipient are replaced by residues from a CDR of a non-human species (donor antibody) such as mouse, rat, or rabbit having the desired specificity, affinity, and capacity. Sometimes, Fv framework residues of the human immunoglobulin are replaced by corresponding non-human residues. Humanized antibodies also comprise residues that are found neither in the recipient antibody nor in the imported CDR or framework sequences. In general, humanized antibodies comprise substantially all of at least one and typically two variable domains wherein all or substantially all of the CDR regions correspond to those of a non-human immunoglobulin and all or substantially all of the FR regions are those of a human immunoglobulin consensus sequence. Humanized antibodies optimally comprise at least a portion of an immunoglobulin constant region (Fc), typically that of a human immunoglobulin.

### Human Antibodies

Human antibodies can be produced using various techniques known in the art, e.g., phage display libraries as described in Hoogenboom and Winter, J. Mol. Biol., 227:381 (1991) and Marks et al., J. Mol. Biol., 222:581 (1991). Human monoclonal antibodies can be produced using the techniques described in Cole et al., Monoclonal Antibodies and Cancer Therapy, Alan R. Liss, p. 77 (1985) and Boemer et al., J. Immunol., 147(1):86-95 (1991). Alternatively, transgenic animals, e.g., mice, are available which, upon immunization, can produce a full repertoire of human antibodies in the absence of endogenous immunoglobulin production. Such transgenic mice are available from Abgenix, Inc., Fremont, California, and Medarex, Inc., Annandale, New Jersey. It has been described that the homozygous deletion of the antibody heavy-chain joining region (JH) gene in chimeric and germ-line mutant mice results in complete inhibition of endogenous antibody production. Transfer of the human germ-tine immunoglobulin gene array in such germ-line mutant mice will result in the production of human antibodies upon antigen challenge. See, e.g., Jakobovits et al., Proc. Natl. Acad. Sci. USA 90:2551 (1993); Jakobovits et al., Nature 362:255-258 (1993); Bruggermann et al., Year in Immunol. 7:33 (1993); and Duchosal et al. Nature 355:258 (1992). Human antibodies can also be derived from phage-display libraries (Hoogenboom et al., J. Mol. Biol. 227:381 (1991); Marks et al., J. Mol. Biol. 222:581-597 (1991); Vaughan, et al., Nature Biotech 14:309(1996)).

### Bispecific Antibodies

Bispecific antibodies can be produced by the recombinant co-expression of two immunoglobulin heavy-chain/light-chain pairs wherein the two heavy chains have different specificities. Bispecific antibodies are monoclonal, preferably human or humanized, antibodies that have binding specificities for at least two different antigens. In the present invention, one of the binding specificities is for the NFAT activating receptor and the other is for any other antigen, preferably a cell surface receptor or receptor subunit. Because of the random assortment of immunoglobulin heavy and light chains, these hybridomas produce a potential mixture of ten different antibodies. However, only one of these antibodies has the correct bispecific structure. The recovery and purification of the correct molecule is usually accomplished by affinity chromatography.

Antibody variable domains with the desired binding specificities (antibody-antigen combining sites) can be fused to immunoglobulin constant domain sequences. The fusion preferably is with an immunoglobulin heavy chain constant domain comprising at least part of the hinge, CH2, and CH3 regions. Preferably, the first heavy-chain constant region (CH1) containing the site necessary for light-chain binding is present in at least one of the fusions. DNAs encoding the immunoglobulin heavy-chain and, if desired, the immunoglobulin light chain is inserted into separate expression vectors and co-transfected into a suitable host organism. Suitable techniques are shown in for producing bispecific antibodies are described in Suresh et al., Methods in Enzymology, 121:210 (1986).

### Heteroconjugate Antibodies

Heteroconjugate antibodies can be produced known protein fusion methods, e.g., by coupling the amine group of one an antibody to a thiol group on another antibody or other polypeptide. If required, a thiol group can be introduced using known methods. For example, immunotoxins comprising an antibody or antibody fragment and a polypeptide toxin can be produced using a disulfide exchange reaction or by forming a thioether bond. Examples of suitable reagents for this purpose include iminothiolate and methyl-4-mercaptobutyrimidate. Such antibodies can be used to target immune complement components and to prevent or treat tissue damage associated with TAAA repair.

The complement inhibitors can be administered to the patient by any means that enables the inhibitor to reach the targeted cells. These methods include, but are not limited to, oral, rectal, nasal, topical, intradermal, subcutaneous, intravenous, intramuscular and intraparenteral modes of administration. Injections are preferred because they permit precise control of the timing and dosage levels used for administration. Preferably the complement inhibitors are administered parenterally. For parenteral administration, the complement inhibitors can be, for example, formulated as a solution, suspension, emulsion or lyophilized powder in association with a physiologically acceptable parenteral vehicle. Examples of such vehicles are water, saline, Ringer's solution, dextrose solution, and 5% human serum albumin. Liposomes and nonaqueous vehicles such as fixed oils may also be used. The vehicle or lyophilized powder may contain additives that maintain isotonicity (e.g., sodium chloride, mannitol) and chemical stability (e.g., buffers and preservatives). The formulation is sterilized by commonly used techniques. For example, a parenteral composition suitable for administration by injection is prepared by dissolving 1.5% by weight of active ingredient in 0.9% sodium chloride solution.

The complement inhibitors can be administered immediately before and/or following an ischemia-reperfusion injury or a TAAA repair, e.g., within 24 hours before and/or within 72 hours following ischemia-reperfusion or a TAAA repair, or can be administered periodically while the patient is recovering from the injury or TAAA repair according to a prescribed dosing schedule, e.g., daily for thirty days, every other day for sixty days, or weekly, designed to minimize treatment frequency and dosage while maximizing the effectiveness of the treatment.

In another aspect, the present invention provides a composition useful for preventing and treating tissue damage associated with ischemia-reperfusion injury or a TAAA repair comprising one or more complement inhibitors and one or more pharmaceutically acceptable adjuvants, carriers, excipients, and/or diluents. Acceptable adjuvants, carriers, excipients, and/or diluents for making pharmaceutical compositions are well known to skilled artisans, e.g., Hoover, John E., Remington's Pharmaceutical Sciences, Mack Publishing Co., Easton, Pennsylvania 1975. Another discussion of drug formulations can be found in Liberman, H. A. and Lachman, L., Eds., Pharmaceutical Dosage Forms, Marcel Decker, New York, N.Y., 1980. Most preferably, the inhibitor is mixed with pharmaceutically acceptable carriers to form a composition that allows for easy dosage preparation and administration. Aqueous vehicles prepared from water having no nonvolatile pyrogens, sterile water, and bacteriostatic water and containing at least 0.025M buffer salts, such as sodium phosphate, sodium bicarbonate, sodium citrate, etc. are also suitable to form injectable complement inhibitor solutions. In addition to these buffers, several other aqueous vehicles can be used. These include isotonic injection compositions that can be sterilized such as sodium chloride, Ringer's, dextrose, dextrose and sodium chloride, and lactated Ringer's. Addition of water-miscible solvents, such as methanol ethanol, or propylene glycol generally increases solubility and stability of the inhibitors in these vehicles. Nonaqueous vehicles such as cottonseed oil, sesame oil, or peanut oil and esters such as isopropyl myristate may also be used as suspension vehicles for the inhibitors. Additionally, various additives which enhance the stability, sterility, and isotonicity of the composition including antimicrobial preservatives, antioxidants, chelating agents, and buffers can be added. Any vehicle, diluent, or additive used would, however, have to be biocompatible and compatible with the inhibitors according to the present invention.
In one embodiment, the composition comprises a first complement inhibitor is selected from the group consisting of anti-MBL antibodies and their functionally equivalent fragments, anti-MASP antibodies and their functionally equivalent fragments, anti-MASP2 antibodies and their functionally equivalent fragments, anti-MASP3 antibodies and their functionally equivalent fragments, and anti-MBL complex antibodies and their functionally equivalent fragment and a second antibody is an antibody different from the first antibody selected from the group consisting of is selected from the group consisting of anti-MBL antibodies and their functionally equivalent fragments, anti-MASP antibodies and their functionally equivalent fragments, anti-MASP2 antibodies and their functionally equivalent fragments, anti-MASP3 antibodies and their functionally equivalent fragments, anti-MBL complex antibodies and their functionally equivalent fragments, and other complement inhibitors. The composition of claim 30 comprising an anti-MBL antibody and one or more different complement inhibitors.

When the complement inhibitor is an antibody or antibody fragment, the formulation is any known formulation suitable for administering antibodies to a patient, e.g., solid antibody formulations such as those disclosed in US Patent Application No. 20020136719, reconstituted lyophilized formulations such as those disclosed in US 6,267,958 or aqueous formulations such as those disclosed in US 6,171,586.

The amount or dosage of complement inhibitor administered to a patient varies depending upon patient type, patient age, patient size, inhibitor type, treatment frequency, administration purpose (therapeutic or prophylactic), and tissue damage severity. Generally, the complement inhibitors are administered to the patient in dosages of from about 2 to 50 milligrams per kilogram of body weight (mg/kg), preferably from about 5 to 30 mg/kg. The complement inhibitors can be administered in one dose or the dose can be broken up into smaller doses that can be administered more frequently. The complement inhibitors can be administered alone or in conjunction to combat tissue damage associated with TAAA repair.

### Examples

This invention can be further illustrated by the following examples of preferred embodiments thereof, although it will be understood that these examples are included merely for purposes of illustration and are not intended to limit the scope of the invention unless otherwise specifically indicated.

### Study Design and Patients

Nineteen thoraco-abdominal aortic aneurysm repair (TAAA) patients above 18 years of age with confirmed diagnosis of TAAA, which extend from the sixth intercostal space to below the renal arteries, or from the diaphragm to below the renal arteries, respectively (Crawford extent III and IV; Coselli JS, LeMarie SA. Surgical techniques: thoracoabdominal aorta. Cardiol Clin North Amer 1999; 4:751-765), undergoing surgical repair were studied. These patients did not need cardiopulmonary bypass, excluding an inflammatory reaction induced by the extracorporeal device. Patients with an abdominal aortic aneurysm or arteriosclerosis undergoing conventional laparatomy (n=5) or endovascular stent graft implantation in the descending aorta (n=6) were included as controls. In the former group visceral ischemia was limited to regions supplied by the inferior mesenteric artery, whereas there was no visceral ischemia in the latter. Exclusion criteria were: respiratory failure requiring ventilator support, kidney failure requiring hemodialysis, shock or severe hypotension. In addition patients with hepatitis and HIV, recent or ongoing systemic bacterial, viral and parasitic infection, history of lupus, rheumatoid arthritis or other diseases which are known to cause elevation of complement were excluded from the study. Preoperative patient demographics for 19 patients undergoing thoraco-abdominal aortic aneurysm repair (TAAA), six patients undergoing endovascular stent grafting of descending aorta (Stent) and five patients undergoing open abdominal aortic aneurysm surgery (Abdominal) are shown in Table 1. During surgery, the patients' routine hemodynamic and respiratory parameters including arterial blood pressure, heart rate, central venous pressure, body temperature, urine output and blood gases were recorded. Clinical information on operation time, clamping time, kidney, visceral and lower extremity ischemic times, blood loss, transfusion, as well as details on postoperative complications and clinical outcome were recorded. Kidneys: Creatinine and blood urea nitrogen. Lungs: PaO₂/FiO₂ ratio, PEEP value, the evaluation of daily chest X-ray, duration of mechanical ventilation, re-intubation. Liver: Total bilirubin, LD, ALT, and AST. Heart: Pressure adjusted heart rate (PAR = HRxCVP/MAP). Blood cells: White blood cell count and platelet count Nervous system: Glasgow Coma Score and manifestations of spinal injury after TAAA repair. Patients were followed up for multi-organ function and postoperative complications until their discharge from the hospital. 30-day mortality was recovered. Six months were spent for enrollment and complete follow-up. In this study the subjects did not undergo any experimental procedures or therapeutic interventions, and no investigational medications were administered.

**Table 1**

| | TAAA | Stent | Abdominal | p-value |
|---|---|---|---|---|
| Age (yrs) | 69 (65-73) | 74 (65-80) | 57 (45-85) | NS¹ |
| Weight (kg) | 73 (65-81) | 65 (53-103) | 70 (50-87) | NS |
| Gender (n) | | | | |
| Male | 11 | 2 | 3 | NS |
| Female | 8 | 4 | 2 | |
| Hypertension | | | | |
| no | 0 | 1 | 2 | NS |
| yes | 19 | 5 | 3 | |
| Hypercholesterolemia | | | | |
| no | 14 | 5 | 2 | NS |
| yes | 5 | 1 | 3 | |
| Diabetes | | | | |
| no | 19 | 5 | 0 | NS |
| yes | 0 | 1 | 0 | |
| Coronary artery disease | | | | |
| no | 8 | 3 | 4 | NS |
| yes | 11 | 3 | 1 | |
| Uremia | | | | |
| no | 19 | 5 | 0 | NS |
| yes | 0 | 1 | 0 | |
| Pulmonary disease | | | | |
| no | 11 | 3 | 4 | NS |
| yes | 8 | 3 | 1 | |
| Cerebrovascular disease | | | | |
| no | 17 | 5 | 4 | NS |
| yes | 2 | 1 | 1 | |
| Smoker | | | | |
| no | 5 | 1 | 1 | NS |
| yes | 14 | 3 | 4 | |
| Peripheral vessel disease | | | | |
| no | 17 | 5 | 1 | p=0.01 |
| yes | 2 | 1 | 4 | |
| Pain | | | | |
| no | 14 | 2 | 5 | NS |
| yes | 5 | 4 | 0 | |
| Etiology | | | | |
| Atherosclerotic | 16 | 4 | 5 | NS |
| Inflammatory | 2 | 2 | 0 | |
| ¹NS = Not statistically significant | | | | |
| Surgical Procedures | | | | |

Both conventional and endovascular procedures were performed in general anesthesia. Each patient received 5-10.000 IU of heparin sodium (Leo-Løvens Kemiske Fabrik, Copenhagen, Denmark) intravenously before aortic clamping or insertion of the endograft. Mild hypothermia (32°C to 34°C, nasopharyngeal) was also used to minimize ischemic complications. Segmental intercostal and lumbar arteries were generally reattached to the graft (Haemashild Gold, Maedox Medicals Inc, NJ, USA). Cerebrospinal fluid (CSF) drainage was not used.) Shed blood was collected with Haemonetcs Cellsaver Device (Haemonetics Corp., Mass, USA) and reinfused in most cases. The renal and visceral arteries were perfused with cold (4°C) crystalloid Ringer acetate (Fresenius Kabi Norge AS, Oslo, Norway) with verapamil (Abbott Laboratories, IL, USA). Neither somatosensory nor motor-evoked potential monitoring was used. Conventional repair of AAA was undertaken by a standard transperitoneal approach involving the use of midline laparotomy and aortic crossclamping below the renal arteries. Endovascular repair was performed with an endograft system (Gore Excluder Thoracic Endoprosthesis, W.L. Gore & Ass, Inc., AZ, USA). Briefly, this modular system consists of a self-expanding stent of braided wires (nitinol) internally covered by ePTFE. Endograft insertion was performed through the common femoral artery, which was occluded for a short period giving distal ischemia.

### Blood and Plasma Transfusions

Red cell concentrates were given to all TAAA patients: 16 patients received median 4 (range 2-9) units, whereas three patients received 17, 30 and 65 units, respectively. 4/6 of patients undergoing endovascular stent grafting and 1/5 of patients undergoing open abdominal surgery received 1-2 units of red cells. Plasma (Octaplas, Octapharma, Vienna, Austria) was given to all TAAA patients except for one (patient A): 17 patients received median 7 (range 4-14) units, whereas one patient received 62 units. None of the patients in the control groups received Octaplas.

### Blood Sampling

Blood samples were obtained at the following time points: T1: Immediately prior to surgery; T2: Prior to aortic clamping; T3: Prior to aortic declamping; T4: Immediately after aortic declamping; T5: 2 hrs after aortic declamping; T6: 8 hrs after aortic declamping; T7: 24 hrs postoperatively; and T8: 72 hrs postoperatively. Venous blood was collected in tubes containing ethylenediaminetetraacetic acid (EDTA), and placed on crushed ice. After immediate centrifugation at +4°C, plasma was collected, and stored at minus 70°C until analysis. Serum was obtained from tubes without anticoagulants after leaving the blood to clot for 2 hrs at room temperature, and stored at minus 70°C.

### Statistics

Due to small sample sizes and non-normal distribution of many variables, data are given as medians with 95 % non-parametric confidence intervals. P-values below 0.05 were considered significant Comparisons among the groups were performed with the X2 test (categorical variables) or Kruskal-Wallis test (continuous variables). Variables measured more than once were first analyzed by two-way repeated measures analysis of variance (ANOVA) using logarithmic or rank transformation if necessary to achieve an appropriate model fit (SPSS-PC program package). If the interaction term was not significant, standard contrast analysis of time-related changes from T1 was performed. Due to differences in the duration of individual operations and the occurrence of non-normal variables and unequal variances, the conditions for two-way repeated measures ANOVA were only partly met. If the interaction term was significant, indicating different changes by time among the groups, subsequent intergroup comparisons were therefore performed with Kruskal-Wallis test and time-related changes within groups were compared with Friedman's non-parametric one-way analysis of variance, which allows for repeated measures. In order to achieve an overall p-value for significance below 0.05 and correct for multiple comparisons, any p-values from such Friedman or Kruskal-Wallis tests below the corresponding p-values from ANOVA were regarded invalid. As a summary measure, the area under the time-curve for the activation parameters was calculated for each patient using known techniques (Altman DG. Practical statistics for medical research. Capman & Hall, 1996) For correlations, Speaman's rank correlation coefficient was calculated. To investigate the relationship between complement activation and postoperative complications in the TAAA patients, the area under the TCC curve was compared between patients experiencing any postoperative complication and patients with an uneventful recovery using Mann-Whitney's U-test.

### Complement Analyses

Mannose-binding lectin (MBL) antigen and function. The concentration of MBL was quantified by a double antibody enzyme-linked immunosorbent assay as follows: A mouse monoclonal anti-human MBL antibody (HYB-131-01, Antibodyshop, Copenhagen, Denmark) was used as capture antibody at 1.0 *µ*g/mL in phosphate buffered saline (PBS) at 4°C overnight. Standard was from the MBL-ELISA (Antibodyshop), giving a lower detection limit of 15 ng/mL. Samples were diluted 1:50 and repeated in dilution 1:10 if lower than 400 ng/mL in first nm. Standards and samples were incubated for one hr at 37°C. A mouse biotinylated monoclonal anti-human MBL (HYB131-01, Antibodyshop) was used as detection antibody at 0.1 *µ*g/mL in PBS containing 0.2% Tween 20, and incubated for one hr at 37°C. Streptavidin-peroxidase and subsequently substrate (ABTS+H₂O₂) was added and optical density read at 410 nm. The function of MBL was measured based on addition of serum to mannan-coated micro-titer wells in high salt concentration to block classical activation and finally detecting deposition of exogenously added C4 (Petersen SV, Thiel S, Jensen L, Steffensen R, Jensenius JC. An assay for the mannan-binding lectin pathway of complement activation. J Immunol Methods 2001; 257(1-2): 107-116). The assay detects the function of MBL as well as the MBL-associated serine proteases (MASPs) in the serum sample.

### Complement Activation Products

The following assays were performed using known methods readily available to scientists: Clrs-Cl-inhibitor complexes (Clrs-Clinh) from the classical pathway (Fure H, Nielsen EW, Hack CE, Mollnes TE. A neoepitope-based enzyme immunoassay for quantification of Cl- inhibitor in complex with Clr and Cls. Scand J Immunol 1997; 46(6):553-557), C4bc reflecting classical as well as mamnose-binding lectin (MBL) pathway (Wolbink GJ, Bollen J, Baars JW, Tenberge RJM, Swaak AJG, Paardekooper J, Hack CE. Application of a monoclonal antibody against a neoepitope on activated C4 in an ELISA for the quantification of complement activation via the classical pathway. J Immunol Methods 1993; 163:67-76), the alternative pathway C3 convertase C3bBbP (Mollnes TE, Brekke OL, Fung M, Fure H, Christiansen D, Bergseth G, Videm V, Lappegard KT, Kohl J, Lambris JD. Essential role of the C5a receptor in E coli-induced oxidative burst and phagocytosis revealed by a novel lepirudin-based human whole blood model of inflammation. Blood 2002; 100(5):1869-1877); C3bc indicating activation of any initial pathway (Garred P, Mollnes TE, Lea T. Quantification in enzyme-linked immunosorbent assay of a C3 neoepitope expressed on activated human complement factor C3. Scand J Immunol 1988; 27:329-335), and the soluble terminal complement complex (TCC) indicating complete activation of the terminal pathway (Mollnes TE, Lea T, Froland SS, Harboe M. Quantification of the terminal complement complex in human plasma by an enzyme-linked immunosorbent assay based on monoclonal antibodies against a neoantigen of the complex. Scand J Immunol 1985; 22:197-202). All assays except C3bBbP are based on monoclonal antibodies recognizing neoepitopes specifically exposed in the activation products and concealed in the native component. The C3bBbP assay is based on detection of properdin (P) bound to C3. Results for all assays are given in arbitrary units (AU)/mL based on fully activated serum (heat aggregated IgG for Clrs-Clinh and C4bc and zymosan for the remaining) defined to contain 1000 AU/mL.

### Cytokines and Chemokines

The following commercial kits were used and the procedure performed according to the manufacture's descriptions to measure the concentration of the following cytokine and chemokines: Interleukin (IL)-1β (DLB50), tumor necrosis factor (TNF)-α (DTA50) and the chemokine IL-8 (D8050) were from R&D Systems, Oxon, UK. IL-6 and IL-10 were from Bender MedSystems, MedSystems Diagnostics GmbH, Vienna, Austria.

### Neutrophil Activation

The neutrophil granula protein myeloperoxidase (MPO) and lactoferrin (LF) were quantified in ELISA using known techniques (Videm V. Heparin in clinical doses primes granulocytes to subsequent activation as measured by myeloperoxidase release. Scand J Immunol 1996; 43(4):385-390 and Hegnhoj J, Schaffalitzky de Muckadell OB. An enzyme linked immunosorbent assay for measurements of lactoferrin in duodenal aspirates and other biological fluids. Scand J Clin Lab Invest 1985; 45(6):489-495).

### Results

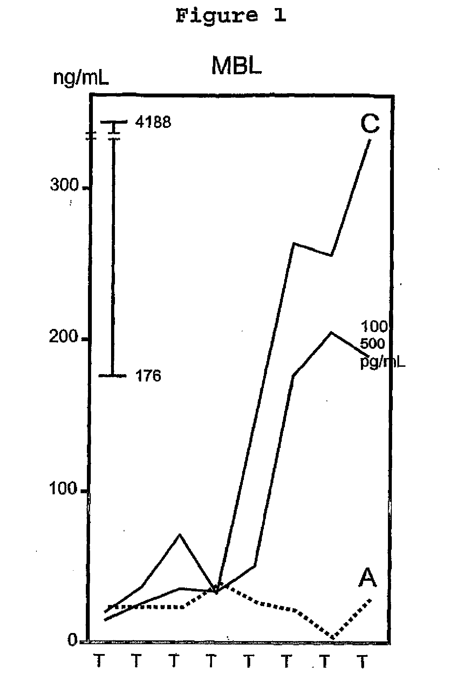

The results from the experiments performed with the nineteen thoraco-abdominal aortic aneurysm repair (TAAA) patients is shown in Figures 1, 2, 3, 4, 5, and 6.

Referring to Figure 1, three patients (A, B and C) undergoing thoracoabdominal aortic aneurysm (TAAA) repair were mannose-binding lectin (MBL) deficient (MBL concentration below 100 ng/mL and undetectable MBL function). Patient A (showed as a dotted line in Figure 1 and the subsequent Figures) did not receive plasma transfusion whereas patient B and C received 5 and 6 units Octaplas, respectively. The MBL concentration range (176-4188) for the 16 MBL-sufficient TAAA patients is indicated by the bar. MBL concentration and function did not change by time in either group. MBL antigen concentration and function correlated significantly (r=0.81, p<0.01). The concentration of MBL at baseline in the TAAA patients did not correlate with the formation of any of the complement activation products. MBL deficiency (antigen level < 100 ng/mL and undetectable function) was found in three of the 19 TAAA patients and in two of the controls. Two of the three TAAA patients (patient B and C) received MBL-containing plasma (Octaplas) transfusion preoperatively whereas one (patient A) did not. Patient B received five units Octaplas between T4 (aortic declamping) and T6 (8 hrs after aortic declamping). Patient C received six units Octaplas between T4 (aortic declamping) and T7 (24 hrs after aortic declamping). Both patient B and C attained plasma MBL concentrations above the lower range of the MBL-sufficient TAAA patients, whereas no change in MBL concentration was seen in patient A who did not receive plasma. Complement activation and inflammatory responses in patient A were strikingly different from the other TAAA patients, but identical to the control patients (no complement activation or increase in IL-1β, TNFα or IL-8, but increase in IL-6 and IL-10), whereas the two MBL-deficient patients receiving plasma (patient B and C) displayed inflammatory responses similar to the MBL-sufficient TAAA patients.

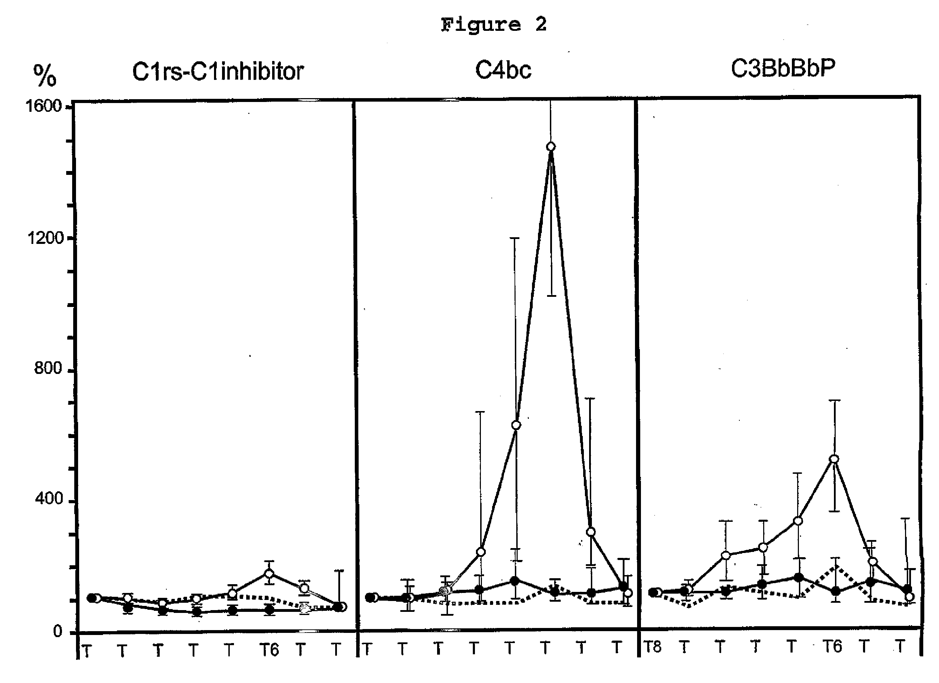

Referring to Figure 2, Clrs-C1 inhibitor complexes (left panel), reflecting classical pathway activation, were slightly increased in the TAAA patients (open circles). In contrast, a substantial increase in C4bc (middle panel), reflecting both classical and lectin pathway activation, and in C3bBbP (right panel), reflecting alternative pathway activation, were seen in the TAAA group. No complement activation was found in the control groups (closed circles indicate the open infrarenal aortic surgery group) or in the MBL-deficient TAAA patient who did not receive plasma (patient A, dotted line). The data (medians and non-parametric 95% confidence intervals) are presented as percent increases from baseline (T1=sample prior to surgery) in order to permit a relative comparison between the initial pathways. Clrs-Clinh increased moderately in the TAAA group from baseline 17 (15-21) to 27 (23-33) AU/mL at 8 hrs after aortic declamping (T6) (p<0.01), whereas no increase was observed in the controls. Due to significant differences at baseline values (T1) among the three groups, the percentage changes from baseline were compared, and the percentage Clrs-Clinh increase in the TAAA group was significantly higher (p<0.01 at T6) than in the controls. C4bc increased markedly in the TAAA group from baseline 6 (5-8) to 89 (74-104) AU/mL at T6 (p<0.01). No increase was observed in the controls and the difference between TAAA and controls was significant (p<0.001 at T6). The relative increase in C4bc (reflecting classical and lectin pathways) was substantially more pronounced than the increase in Clrs-Clinh (classical pathway only). C3bBbP (alternative pathway) increased in the TAAA group from baseline 11 (7-17) to 47 (36-65) AU/mL at T6 (p<0.01). No increase was observed in the controls and the difference between TAAA and controls was significant (p<0.001 at T6).

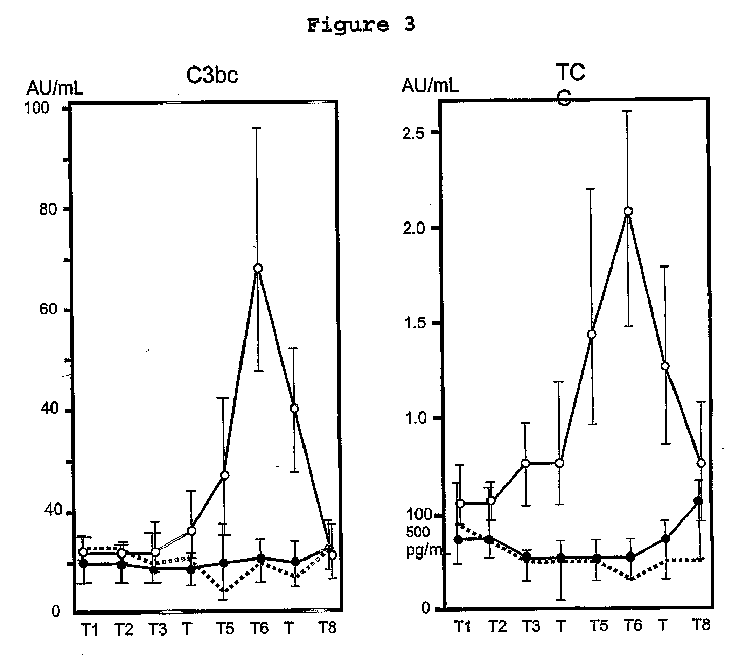

Referring to Figure 3, C3bc (for all pathways) (left panel) increased in the TAAA group from baseline 12 (8-15) to 69 (48-96) AU/mL at T6 (p<0.01). No increase was observed in the controls and the difference between TAAA (open circles) and controls (closed circles) was significant (p<0.001 at T6). TCC (terminal pathway) increased in the TAAA group from baseline 0.6 (0.5-0.8) to 2.1 (1.5-2.6) AU/mL at T6 (p<0.05). No increase was observed in the controls and the difference between TAAA and controls was significant (p<0.01 at T6). All complement activation products reached a maximum at 8 hrs after aortic declamping and thereafter declined. In the MBL-deficient TAAA patient (patient A, dotted line) who did not receive plasma transfusion, there were no increases in any of the activation products. Data are medians and non-parametric 95% confidence intervals.

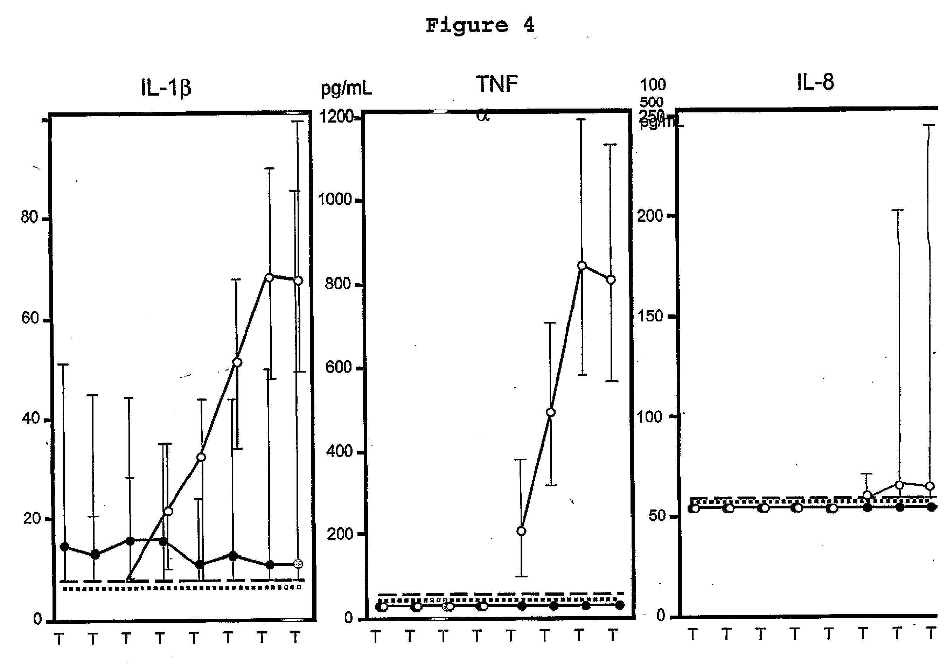

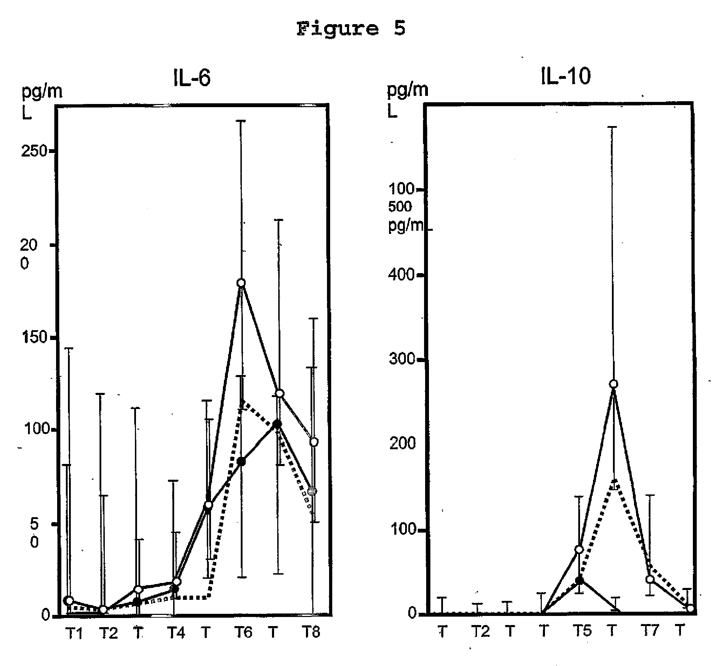

Referring to Figures 4 and 5, two distinct activation patterns were revealed: IL-1β, TNFα and IL-8 increased in the TAAA group only, reached a peak at 24 hrs after aortic declamping (T7) and were closely correlated to the degree of complement activation. IL-6 and IL-10, on the other hand, reached a maximum at 8 hrs after aortic declamping (T6) in the TAAA group (open circles), were not correlated to the degree of complement activation and increased also in the control groups (closed circles). IL-1β increased in the TAAA group from baseline < 8 (<8-9) (8 = lower detection limit) to 69 (48-90) pg/mL at 24 hrs after aortic declamping (T7) (p<0.0001) whereas no increase was observed in the controls. TNFα increased in the TAAA group from baseline < 78 (lower detection limit) to 868 (603-1210) pg/mL at T7 (p<0.0001), whereas no increase was observed in the controls. IL-8 increased in 10 of the 19 TAAA patients from baseline < 63 (lower detection limit) to 70 (<63-207) pg/ml at T7 (p<0.0001), whereas no increase was seen in the controls. The maximum increases in IL-1β, TNFα and IL-8 occurred later (24 hrs after aortic declamping) than maximum complement activation (8 hrs after aortic declamping). The degree of complement activation, as measured by the area under the TCC curve, was significantly correlated with the areas under the IL-1β (r=0.66; p=0.007), TNFα (r=0.68; p=0.006) and IL-8 (r=0.81; p<0.0005) curves. The MBL deficient TAAA patient (patient A, dotted line) who did not receive plasma transfusion (patient A) had undetectable levels of both TNFα, IL-1β and IL-8. IL-6 increased in the TAAA, group from baseline 6 (3-19) to maximum 186 (114-271) pg/mL at 8 hrs after aortic declamping (T6) (p<0.0001). IL-6 also increased significantly in the control groups although less extensively and showing later timepoints for maximal concentrations: from baseline 13 (3-150) to 108 (27-122) pg/mL at 24 hrs postoperatively (T7) in the laparatomy group (p<0.05) and from baseline 8 (3-86) to 81 (33-131) pg/mL at 72 hrs postoperatively (T8) in the endovascular group (p=0.001). IL-10 increased in the TAAA group from baseline 8 (7-9) to maximum 281 (156-581) pg/mL at 8 hrs after aortic declamping (T6) (p=0.01). IL-10 increased only slightly in the control groups: from baseline 6 (6-7) to 35 (7-83) pg/mL at 2 hrs after aortic declamping (T5) (p=0.01) in the laparotomy group and from baseline 8 (6-17) to 15 (8-27) pg/mL at 72 hrs after aortic declamping (T8) in the endovascular group (p=0.01). Notably, IL-6 and IL-10 peaked at the same time as the complement activation products in the TAAA group (8 hrs after aortic declamping), but there was no correlation between complement activation (area under the TCC curve) and areas under the IL-6 (r=0.32; p=0.18) or IL-10 (r=0.20; p=0.42) curves. Both IL-6 and IL-10 increased in the MBL deficient TAAA patient who did not receive plasma (patient A, dotted line), comparable to the controls.

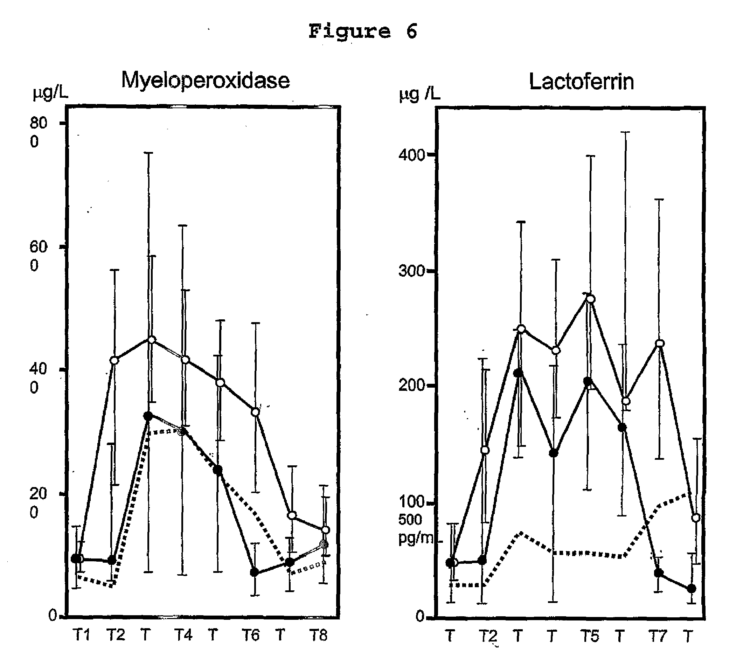

Referring to Figure 6, although the increase in MPO and lactoferrin occurred prior to complement activation, there was a significant correlation between complement activation (area under the curve for TCC) and the areas under the MPO (r=0.70; p=0.001) and LF (r=0.63; p=0.004) curves, indicating a complex pattern of neutrophil activation during TAAA repair. MPO (left panel) and lactoferrin (right panel) increased early (T3=before declamping) in all groups, slightly more in the TAAA group (open circles), than in the controls (closed circles indicate the open infrarenal aortic surgery group). Dotted line indicates the MBL-deficient TAAA patient who did not receive plasma (patient A, dotted line). Data are medians and non-parametric 95% confidence intervals.

### Plasma Transfusions

To exclude plasma transfusions as a source of inflammatory markers, Octaplas was tested for cytokines and found to contain undetectable levels. Furthermore, no correlation was found between the number of plasma transfusions and changes in complement activation products, cytokines or neutrophil granula proteins (p=0.13-0.63).

### Clinical Outcome

Perioperative clinical data was collected for the nineteen patients undergoing thoraco-abdominal aortic aneurysm repair (TAAA), six patients undergoing endovascular stent grafting of descending aorta (Stent), and five patients undergoing open abdominal aortic aneurysm surgery (Abdominal). The results are shown in Table 2.

**Table 2**

| | | TAAA | Stent | Abdominal | p-value |
|---|---|---|---|---|---|
| Skin-skin time (min) | | 164 (140-190) | 100 (61-231) | 189 (132-233) | NS¹ |
| Visceral ischemia (min) | | 37 (30-44) | | | |
| Lower extr.ischemia (min) | | 54 (44-63) | 73 (40-116) | 72(45-98) | NS |
| Total ischemia (min) | | 55(46-65) | 73 (40-116) | 72(40-116) | NS |
| Graft size (mm) | | 23 (21-24) | 31 (29-38) | 15(14-16) | <0.001 |
| Graft type (n) | | | | | |
| | bifurcated | 10 | 0 | 2 | |
| | straight | 9 | 6 | 1 | |
| | endarterectomy | 0 | 0 | 2 | |
| ICU stay (days) | | 1(1-2) | 1 (all) | 0 (0-3) | 0.04 |
| Respirator (hrs) | | 11 (8-26) | | | |
| Infusions (mL) | | 3800 | 3050 | 7400 | 0.004 |
| | | (3100-4500) | (1097-5947) | (6250-8250) | |

| | | | | | |
|---|---|---|---|---|---|
| ¹NS =Not statistically significant | | | | | |

Mortality and postoperative complications were observed in 19 patients undergoing thoraco-abdominal aortic aneurysm repair (TAAA), six patients undergoing endovascular stent grafting of descending aorta (Stent) and five patients undergoing open abdominal aortic aneurysm surgery (Abdominal). The results are shown in Table 3.

**Table 3**

| | | TAAA | Stent | Abdominal | p-value |
|---|---|---|---|---|---|
| Mortality | | 1 | 0 | 0 | NS¹ |
| Postoperative | | | | | |
| complication | | | | | |
| | No | 10 | 5 | 4 | NS |
| | Yes | 9 | 1 | 1 | |
| Inotropy | | | | | |
| | No | 11 | 5 | 5 | NS |
| | Yes | 8 | 1 | 0 | |
| Reintubation | | 2 | 0 | 0 | NS |
| | Reoperation | 3 | 0 | 0 | NS |
| | Thromboembolism | 1 | 0 | 0 | NS |
| | Myocardial infarction | 2 | 0 | 0 | NS |
| | Uremia | 5 | 1 | 0 | NS |
| | Gastrointestinal failure | 2 | 1 | 0 | NS |
| | Multiple organ failure | 3 | 0 | 0 | NS |
| | Wound infection | 2 | 1 | 0 | NS |
| | Other complications | 4 | 1 | 0 | NS |

| | | | | | |
|---|---|---|---|---|---|
| ¹NS = Not statistically significant | | | | | |

Referring to Tables 2 and 3, one patient in the TAAA group died the first postoperative day due to massive bleeding and bowl ischemia. The study is too small to allow statistical comparison of clinical parameters among the groups. However, when complications were grouped as "yes" (n=9) or "no" (n=10) for each individual patient, there was a trend towards higher degree of complement activation (larger areas under the TCC curve) for patients in the TAAA group experiencing complications (2625 (1760-3345) AU/mL) than for those without (1514 (951-2210) AU/mL) (p=0.06).

The data from the examples show that complement activation is an indicator for severity of clinical complications in patients undergoing TAAA repair and that this activation is mainly mediated by the lectin pathway and amplified through the alternative pathway. Therefore, methods for inhibiting or preventing complement activation are useful for preventing and treating tissue damage associated with TAAA repair.

In the specification, there have been disclosed typical preferred embodiments of the invention and, although specific terms are employed, they are used in a generic and descriptive sense only and not for purposes of limitation, the scope of the invention being set forth in the following claims. Obviously many modifications and variations of the present invention are possible in light of the above teachings. It is therefore to be understood that within the scope of the appended claims the invention may be practiced otherwise than as specifically described. Preferred embodiments of the present invention are disclosed below and are designated as embodiments E1 to E33:
E1. A method for preventing or treating tissue damage associated with ischemia-reperfusion injury comprising
   administering a tissue damaging preventing or treating amount of one or more complement inhibitors to a patient likely to suffer from or suffering from tissue damage associated with ischemia-reperfusion injury.
E2. The method of E1 wherein the complement inhibitor is selected from the group consisting of compstatin and its functional analogs, C1 Inhibitor, sCR1 and its analogues, anti-C5 antibodies and their functionally equivalent fragments, anti-C5a antibodies and their functionally equivalent fragments, anti-C5a receptor antibodies and their functionally equivalent fragments, anti-C3a antibodies and their functionally equivalent fragments, anti-C3a receptor antibodies and their functionally equivalent fragments, anti-C6 antibodies and their functionally equivalent fragments, anti-C7 antibodies and their functionally equivalent fragments, anti-C8 antibodies and their functionally equivalent fragments, anti-C9 antibodies and their functionally equivalent fragments, anti-properdin antibodies and their functionally equivalent fragments, fusion protein Membrane Cofactor Protein (MCP), Decay Accelerating Factor (DAF), clusterin, CD59, C4bp, Factor H, Factor I, Carboxypeptidase N, vitronectin (S Protein), SP-40, CD59, and Homologous Restriction Factor (HRF).
E3. The method of E1 wherein the complement inhibitor inhibits a component of the lectin pathway.
E4. The method of E3 wherein the complement inhibitor is selected from the group consisting of anti-MBL antibodies and their functionally equivalent fragments, anti-MASP1 antibodies and their functionally equivalent fragments, anti-MASP2 antibodies and their functionally equivalent fragments, anti-MASP3 antibodies and their functionally equivalent fragments, anti-MBL complex antibodies and their functionally equivalent fragments, mannan binding lectin receptor antagonists, keratin binding molecules, anti-keratin antibodies and their functionally equivalent fragments, MASP1 binding peptides, MASP2 binding peptides, and MASP3 binding peptides.
E5. The method of E1 wherein the complement inhibitor is administered to the patient in dosages of from about 2 to 50 milligrams per kilogram of body weight.
E6. The method of E1 wherein the complement inhibitor is an antibody or a functionally equivalent fragment thereof.
E7. The method of E1 wherein the antibody inhibits a component of the lectin complement pathway.
E8. The method of E7 wherein the antibody is selected from the group consisting of anti-MBL antibodies and their functionally equivalent fragments, anti-MASP1 antibodies and their functionally equivalent fragments, anti-MASP2 antibodies and their functionally equivalent fragments, anti-MASP3 antibodies and their functionally equivalent fragments, and anti-MBL complex antibodies and their functionally equivalent fragments.
E9. The method of E7 wherein the antibody is an anti-MBL antibody or functionally equivalent fragments thereof.
E10. The method of E1 wherein at least two complement inhibitors are administered to the patient.
E11. The method of E10 wherein the complement inhibitors are administered in conjunction to the patient.
E12. The method of E10 wherein one complement inhibitor is an anti-MBL antibody.
E13. The method of E1 wherein the complement inhibitor is administered within 24 hours before or within 72 hours after a patient suffers an ischemia-reperfusion injury.
E14. The method of claim 1 wherein the complement inhibitor is administered periodically after a patient suffers an ischemia-reperfusion injury.
E15. A method for preventing or treating tissue damage associated with thoraco-abdonimal aortic aneurysm repair comprising administering a tissue damaging preventing or treating amount of one or more complement inhibitors to a patient likely to suffer from or suffering from tissue damage associated with thoraco-abdominal aortic aneurysm repair.
E16. The method of E15 wherein the complement inhibitor is selected from the group consisting of compstatin and its functional analogs, C1 Inhibitor, sCR1 and its analogues, anti-C5 antibodies and their functionally equivalent fragments, anti-C5a antibodies and their functionally equivalent fragments, anti-C5a receptor antibodies and their functionally equivalent fragments, anti-C3a antibodies and their functionally equivalent fragment, anti-C3a receptor antibodies and their functionally equivalent fragments, anti-C6 antibodies and their functionally equivalent fragments, anti-C7 antibodies and their functionally equivalent fragments, anti-C8 antibodies and their functionally, equivalent fragments, anti-C9 antibodies and their functionally equivalent fragments, anti-properdin antibodies and their functionally equivalent fragments, fusion protein Membrane Cofactor Protein (MCP), Decay Accelerating Factor (DAF), clusterin, CD59, C4bp, Factor H, Factor I, Carboxypeptidase N, vitronectin (S Protein), SP-40, CD59, and Homologous Restriction Factor (HRF).
E17. The method ofE 15 wherein the complement inhibitor inhibits a component of the lectin pathway.
E18. The method of E17 wherein the complement inhibitor is selected from the group consisting of anti-MBL antibodies and their functionally equivalent fragments, anti-MASP1 antibodies and their functionally equivalent fragments, anti-MASP2 antibodies and their functionally equivalent fragments, anti-MASP3 antibodies and their functionally equivalent fragments, anti-MBL complex antibodies and their functionally equivalent fragments, mannan binding lectin receptor antagonists, keratin binding molecules, anti-keratin antibodies and their functionally equivalent fragments, MASP1 binding peptides, MASP2 binding peptides, and MASP3 binding peptides.
E19. The method of E15 wherein the complement inhibitor is administered to the patient in dosages of from about 2 to 50 milligrams per kilogram of body weight.
E20. The method of E15 wherein the complement inhibitor is an antibody or a functionally equivalent fragment thereof.
E21. The method of E15 wherein the antibody inhibits a component of the lectin complement pathway.
E22. The method of E21 wherein the antibody is selected from the group consisting of anti-MBL antibodies and their functionally equivalent fragments, anti-MASP1 antibodies and their functionally equivalent fragments, anti-MASP2 antibodies and their functionally equivalent fragments, anti-MASP3 antibodies and their functionally equivalent fragments, and anti-MBL complex antibodies and their functionally equivalent fragments.
E23. The method of E21 wherein the antibody is an anti-MBL antibody or functionally equivalent fragments thereof.
E24. The method of E15 wherein at least two complement inhibitors are administered to the patient.
E25. The method of E24 wherein the complement inhibitors are administered in conjunction to the patient.
E26. The method of E24 wherein one complement inhibitor is an anti-MBL antibody.
E27. The method of E15 wherein the complement inhibitor is administered within 24 hours before or within 72 hours after a patient suffers an ischemia-reperfusion injury.
E28. The method of E15 wherein the complement inhibitor is administered periodically after a patient suffers an ischemia-reperfusion injury.
E 29. A composition useful for the prevention and treatment of tissue damage associated with an ischemia-reperfusion injury or a thoraco-abdominal aortic aneurysm repair comprising one or more complement inhibitors and one or more pharmaceutically acceptable adjuvants, carriers, excipients, and diluents.
E 30. The composition of E29 comprising two or more complement inhibitors.
E31. The composition of E30 wherein one of the complement inhibitors is an antibody or functionally equivalent fragment thereof.
E32. The composition of E 30 wherein a first complement inhibitor is selected from the group consisting of anti-MBL antibodies and their functionally equivalent fragments, anti-MASP antibodies and their functionally equivalent fragments, anti-MASP2 antibodies and their functionally equivalent fragments, anti-MASP3 antibodies and their functionally equivalent fragments, and anti-MBL complex antibodies and their functionally equivalent fragments and a second antibody different from the first antibody is selected from the group consisting of anti-MBL antibodies and their functionally equivalent fragments, anti-MASP antibodies and their functionally equivalent fragment, anti-MASP2 antibodies and their functionally equivalent fragments, anti-MASP3 antibodies and their functionally equivalent fragment, anti-MBL complex antibodies and their functionally equivalent fragments, and other complement inhibitors.
E33. The composition of E31 comprising an anti-MBL antibody or functionally equivalent fragment thereof and one or more different complement inhibitors.

## Claims

1. A method for preventing or treating tissue damage associated with thoraco-abdominal aortic aneurysm repair comprising administering a tissue damaging preventing or treating amount of one or more complement inhibitors to a patient likely to suffer from or suffering from tissue damage associated with thoraco- abdominal aortic aneurysm repair.

2. The method of claim 1 wherein the complement inhibitor is compstatin or one of its functional analogs, a C1 Inhibitor, sCR1 or one of its analogues, an anti-C5 antibody or functionally equivalent fragment thereof, an anti-C5a antibody or functionally equivalent fragment thereof, an anti-C5a receptor antibody or functionally equivalent fragment thereof, an anti-C3a antibody or functionally equivalent fragment thereof, an anti-C3a receptor antibody or functionally equivalent fragment thereof, an anti-C6 antibody or functionally equivalent fragment thereof, an anti-C7 antibody or functionally equivalent fragment thereof, an anti-C8 antibody or functionally equivalent fragment thereof, an anti-C9 antibody or functionally equivalent fragment thereof, an anti-properdin antibody or functionally equivalent fragment thereof, a fusion protein Membrane Cofactor Protein (MCP), a Decay Accelerating Factor (DAF), clusterin, CD59, C4bp, Factor H, Factor I, Carboxypeptidase N, vitronectin (S Protein), SP-40, CD59 or Homologous Restriction Factor (HRF).

3. The method of claim 1 wherein the complement inhibitor inhibits a component of the lectin pathway, and is an anti-MBL antibody or functionally equivalent fragment thereof, an anti-MASP1 antibody or functionally equivalent fragment thereof, an anti-MASP2 antibody or functionally equivalent fragment thereof, an anti-MASP3 antibody or functionally equivalent fragment thereof, an anti-MBL complex antibody or functionally equivalent fragment thereof, a mannan binding lectin receptor antagonist, a keratin binding molecule, an anti-keratin antibody or functionally equivalent fragment thereof, a MASP1 binding peptide, a MASP2 binding peptide or a MASP3 binding peptide.

4. The method of claim 1 wherein the complement inhibitor is administered to the patient in dosages of from about 2 to 50 milligrams per kilogram of body weight.

5. The method of claim 1 wherein the complement inhibitor is an antibody or a functionally equivalent fragment thereof.

6. The method of claim 5 wherein the antibody inhibits a component of the lectin complement pathway, and is an anti-MBL antibody or functionally equivalent fragment thereof, an anti-MASP1 antibody or functionally equivalent fragment thereof, an anti-MASP2 antibody or functionally equivalent fragment thereof, an anti-MASP3 antibody or functionally equivalent fragment thereof or an anti-MBL complex antibody or functionally equivalent fragment thereof.

7. The method of claim 6 wherein the antibody is an anti-MBL antibody or functionally equivalent fragment thereof.

8. The method of claim 1 wherein at least two complement inhibitors are administered to the patient, optionally wherein said complement inhibitors are administered in conjunction to the patient.

9. The method of claim 8 wherein one complement inhibitor is an anti-MBL antibody.

10. The method of claim 1 wherein the complement inhibitor is administered:
(i) within 24 hours before or within 72 hours after a patient suffers an ischemia-reperfusion injury; or
(ii) periodically after a patient suffers an ischemia-reperfusion injury.

11. A composition useful for the prevention and treatment of tissue damage associated with a thoraco-abdominal aortic aneurysm repair comprising one or more complement inhibitors and one or more pharmaceutically acceptable adjuvants, carriers, excipients, and diluents.

12. The composition of claim 11 comprising two or more complement inhibitors.

13. The composition of claim 12 wherein one of the complement inhibitors is an antibody or functionally equivalent fragment thereof.

14. The composition of claim 12 wherein a first complement inhibitor is an anti-MBL antibody or functionally equivalent fragment thereof, an anti-MASP1 antibody or functionally equivalent fragment thereof, an anti-MASP2 antibody or functionally equivalent fragment thereof, an anti-MASP3 antibody or functionally equivalent fragment thereof or an anti-MBL complex antibody or functionally equivalent fragment thereof and a second antibody different from the first antibody is an anti-MBL antibody or functionally equivalent fragment thereof, an anti-MASP1 antibody or functionally equivalent fragment thereof, an anti-MASP2 antibody or functionally equivalent fragment thereof, an anti-MASP3 antibody or functionally equivalent fragment thereof, an anti-MBL complex antibody or functionally equivalent fragment thereof or other complement inhibitors.

15. The composition of claim 13 comprising an anti-MBL antibody or functionally equivalent fragment thereof and one or more different complement inhibitors.
